# EUROPEAN PATENT APPLICATION

(11) **EP 4 394 386 A1**
(43) Date of publication of application: **03.07.2024**
(21) Application number: 22904163.7
(22) Date of filing: 02.12.2022
(51) Int. Cl.: G01N 35/00

(54) **AUTOMATIC ANALYSIS DEVICE AND REAGENT AMOUNT DISPLAY METHOD**

(30) Priority: 09.12.2021 JP 2021199980
(71) Applicant: Sekisui Medical Co., Ltd., Tokyo 103-0027 (JP)
(72) Inventor: KAWABE, Toshiki, Tokyo 103-0027 (JP); IIJIMA, Yumi, Tokyo 103-0027 (JP); OGASAWARA, Kosuke, Tokyo 103-0027 (JP)
(74) Representative: Schäfer, Matthias W.
(86) International application number: PCT/JP2022/044574
(87) International publication number: WO 2023/106234

(57) **Abstract**

Provided are an automatic analysis device and a reagent amount display method capable of setting an appropriate amount of reagent at all times regardless of a skill level of an operator.

The automatic analysis device of the invention includes a history information storage unit 80 configured to store history information related to a previously conducted analysis, a number-of-analyses calculation unit 81 configured to calculate a number of analyses per unit, which is a number of analyses per day on each analysis day, for each analysis item from the history information, a classification/storage unit 82 configured to classify and store the number of analyses per unit by day of the week, a reagent remaining amount detection unit 83 configured to detect a reagent remaining amount, a conversion unit 84 configured to read each number of analyses per unit over a predetermined period corresponding to a day of a week of an analysis implementation date from the classification/storage unit 82 and convert the read each number of analyses per unit into a reagent usage amount with regard to each reagent used for the analysis for each analysis item, and a display device 192 configured to arrange and display information associated with the reagent usage amount and the reagent remaining amount for each reagent.

## Description

### TECHNICAL FIELD

The present invention relates to an automatic analysis device including a reaction unit that holds a reaction vessel into which a sample collected from a person such as blood or urine is dispensed, and a reagent supply unit that supplies a reagent, and obtaining measurement information with regard to a predetermined analysis item by measuring a test liquid obtained by mixing and reacting the sample and the reagent supplied from the reagent supply unit to the reaction vessel, and a reagent amount display method in the automatic analysis device.

### BACKGROUND ART

Various forms have conventionally been known as an automatic analysis device capable of obtaining measurement information with regard to various analysis items by measuring a test liquid obtained by mixing and reacting a biological sample such as blood or urine with various reagents, such as a blood coagulation analysis device and an analysis device using immunoassay. For example, a sample as a biological sample is dispensed from a sample vessel to a reaction vessel, and a reagent corresponding to an analysis item is mixed with the dispensed sample to perform various measurements and analyses (for example, see Patent Document 1).

### CITATION LIST

### PATENT DOCUMENT

Patent Document 1: JP 2015-057614 A

### SUMMARY OF THE INVENTION

### PROBLEM TO BE SOLVED BY THE INVENTION

Incidentally, in analyses conducted at actual testing sites (hospitals, testing centers, etc.), the number of measurements for each analysis item varies depending on the day of the week, and in general testing sites, there is a certain tendency in the number of measurements depending on the day of the week. An operator who is familiar with such trends can set the appropriate amount of reagent in the reagent supply unit depending on the day of the week. However, an inexperienced operator cannot know the number of measurements for each day of the week, and thus does not know the optimal amount of reagent to be preset in the automatic analysis device before starting analysis. Therefore, when the amount of reagent provided in the automatic analysis device is insufficient, the reagent runs out during analysis to force the analysis to be interrupted, thereby causing inconvenience such as wasting the sample being analyzed. Further, to avoid such a situation, it is conceivable to set a reagent in the automatic analysis device with a considerable margin. However, in this case, even on days of the week when the number of analyses tends to be small, a large number of reagents are set, which frequently results in surplus reagents. Once a normal reagent is opened, a period of use from that time point on is limited, which can be said to be inefficient reagent operation.

The invention has been made with attention to the above-mentioned problems, and an object of the invention is to provide an automatic analysis device and a reagent amount display method capable of setting an appropriate amount of reagent at all times regardless of a skill level of an operator.

### MEANS FOR SOLVING PROBLEM

To achieve the above objects, the invention is an automatic analysis device including a reaction unit configured to hold a reaction vessel into which a sample collected from a person such as blood or urine is dispensed, and a reagent supply unit configured to supply a reagent, and obtaining measurement information with regard to a predetermined analysis item by measuring a test liquid obtained by mixing and reacting the reagent supplied from the reagent supply unit to the reaction vessel with the sample, the automatic analysis device including a history information storage unit configured to store history information related to a previously conducted analysis, a number-of-analyses calculation unit configured to calculate a number of analyses per unit, the number of analyses per unit being a number of analyses per day on each analysis day, for each analysis item from the history information stored in the history information storage unit, a classification/storage unit configured to classify the number of analyses per unit calculated by the number-of-analyses calculation unit for each day of a week and store the number of analyses per unit, a reagent remaining amount detection unit configured to detect a current remaining amount of each reagent in the reagent supply unit, a conversion unit configured to read each number of analyses per unit over a predetermined period corresponding to a day of a week of an analysis implementation date from the classification/storage unit and convert the read each number of analyses per unit into a reagent usage amount with regard to each reagent used for the analysis for each analysis item, and a display unit configured to arrange and display information associated with each reagent usage amount converted by the conversion unit and a remaining amount of each reagent detected by the reagent remaining amount detection unit for each reagent.

In addition, the invention is a reagent amount display method in an automatic analysis device including a reaction unit configured to hold a reaction vessel into which a sample collected from a person such as blood or urine is dispensed, and a reagent supply unit configured to supply a reagent, and obtaining measurement information with regard to a predetermined analysis item by measuring a test liquid obtained by mixing and reacting the reagent supplied from the reagent supply unit to the reaction vessel with the sample, the method including a history information storage step of storing history information related to a previously conducted analysis, a number-of-analyses calculation step of calculating a number of analyses per unit, the number of analyses per unit being a number of analyses per day on each analysis day, for each analysis item from the history information stored in the history information storage step, a classification/storage step of classifying the number of analyses per unit calculated in the number-of-analyses calculation step for each day of a week and storing the number of analyses per unit in a storage unit, a reagent remaining amount detection step of detecting a current remaining amount of each reagent in the reagent supply unit, a conversion step of reading each number of analyses per unit over a predetermined period corresponding to a day of a week of an analysis implementation date from the classification/storage unit and converting the read each number of analyses per unit into a reagent usage amount with regard to each reagent used for the analysis for each analysis item, and a display step of arranging and displaying information associated with each reagent usage amount converted by the conversion step and a remaining amount of each reagent detected by the reagent remaining amount detection step for each reagent.

According to the automatic analysis device having the above configuration and the display method, the number of analyses per unit, which is the number of analyses per day on each analysis day, is calculated for each analysis item from the history information related to the previously conducted analysis, the calculated number of analyses per unit is classified by day of the week, each number of analyses per unit over the predetermined period corresponding to the day of the week of the implementation analysis date is converted into the reagent usage amount with regard to each reagent used for the analysis for each analysis item, and information related to the converted each reagent usage amount and the current remaining amount of each reagent in the reagent supply unit is arranged and displayed for each reagent. Therefore, according to this display, even an inexperienced operator easily understands a difference between the estimated (or required) reagent amount necessary on the day (day of the week) and the reagent amount previously provided in the automatic analysis device. Therefore, an appropriate amount of reagent can be maintained at all times by replenishment with at least the reagent of the amount corresponding to the difference before starting to analyze the analysis implementation date. For this reason, it is possible to avoid inconvenience of running out of reagent during analysis to force the analysis to be interrupted, thereby causing such as wasting the sample being analyzed. Further, such a display mode using the automatic analysis device itself eliminates the need for the automatic analysis device to be connected to a central management system that can accumulate and calculate analysis data, and is effective in facilities where there are few skilled laboratory technicians.

In addition, based on such parallel display of the information associated with the reagent usage amount and the reagent remaining amount, the operator only needs to perform replenishment with the minimum amount of reagent required before starting the analysis on the analysis implementation date, and thus there is no need to set a reagent in the automatic analysis device with a considerable margin. Therefore, it is also possible to avoid inefficient reagent operation due to the above-mentioned restriction on the reagent usage period resulting from excess reagent.

### EFFECT OF THE INVENTION

According to the invention, it is possible to provide an automatic analysis device and a reagent amount display method capable of setting an appropriate amount of reagent at all times regardless of a skill level of an operator.

### BRIEF DESCRIPTION OF DRAWINGS

Fig. 1 is a schematic overall external view of an automatic analysis device according to an embodiment of the invention;
Fig. 2 is a schematic plan view illustrating an internal configuration of the automatic analysis device of Fig. 2;
Fig. 3 is a block diagram of a configuration for realizing a reagent amount display method according to an embodiment of the invention;
Fig. 4 is a flowchart illustrating an example of steps of the reagent amount display method according to an embodiment of the invention;
Fig. 5 is a diagram illustrating a first example of a reagent amount display mode by a display device;
Fig. 6 is a diagram illustrating a second example of the reagent amount display mode by the display device;
Fig. 7 is a diagram illustrating a third example of the reagent amount display mode by the display device; and
Fig. 8 is a diagram illustrating a fourth example of the reagent amount display mode by the display device.

### MODE(S) FOR CARRYING OUT THE INVENTION

Embodiments of the invention will be described below with reference to the drawings.

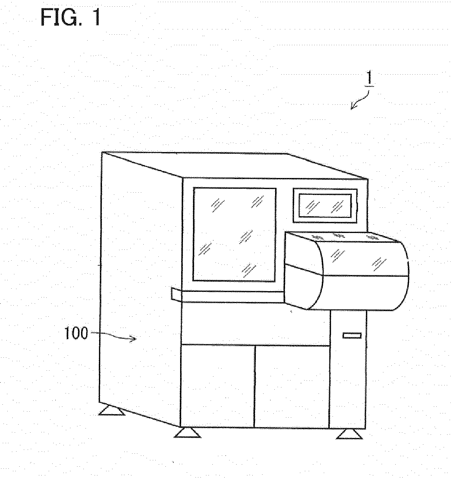

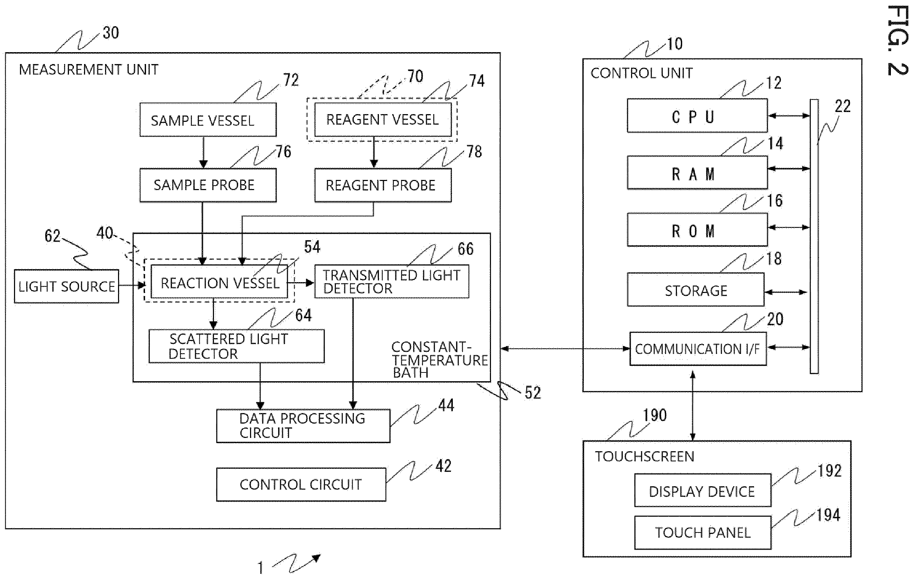

Fig. 1 is a schematic overall external view of an automatic analysis device of this embodiment, and Fig. 2 is a block diagram illustrating a schematic internal configuration of the automatic analysis device of Fig. 1. As illustrated in Fig. 2, the automatic analysis device 1 of this embodiment includes a reaction unit 40 that holds a reaction vessel 54 into which a sample collected from a person such as blood or urine is dispensed, and a reagent supply unit 70 that supplies a reagent in a reagent vessel 74 to the reaction vessel 54, and obtains measurement information with regard to a predetermined analysis item (test item) by reacting the reagent supplied from the reagent supply unit 70 to the reaction vessel 54 with the sample to measure a reaction progress (measure a progress of a test liquid obtained by mixing and reacting the reagent with the sample).

Specifically, the automatic analysis device 1 of this embodiment has an outer frame formed by a casing 100, and is configured by forming a sample processing space in an upper part in the casing 100 (see Fig. 1).

As clearly illustrated in Fig. 2, the automatic analysis device 1 includes a control unit 10, a measurement unit 30 and a display/operation unit. In this embodiment, for example, a touchscreen 190 is provided as the display/operation unit.

The control unit 10 controls the entire operation of the automatic analysis device 1. The control unit 10 includes, for example, a personal computer (PC). The control unit 10 includes a Central Processing Unit (CPU) 12, a Random Access Memory (RAM) 14, a Read Only Memory (ROM) 16, a storage 18, and a communication interface (I/F) 20, which are connected to each other via a bus line 22. The CPU 12 performs various signal processing, etc. The RAM 14 functions as a main storage device of the CPU 12. For example, a Dynamic RAM (DRAM), a Static RAM (SRAM), etc. can be used as the RAM 14. The ROM 16 records various startup programs, etc.

For example, a hard disk drive (HDD), a solid state drive (SSD), etc. can be used as the storage 18. Various information such as programs and parameters used by the CPU 12 are recorded in the storage 18. Furthermore, data acquired by the measurement unit 30 is recorded in the storage 18. The RAM 14 and the storage 18 are not limited thereto, and may be replaced with various storage devices. The control unit 10 communicates with external devices such as the measurement unit 30 and the touchscreen 190 via the communication I/F 20.

The touchscreen 190 includes a display device 192 as a display unit and, for example, a touch panel 194 as an operation unit. The display device 192 may include, for example, a liquid crystal display (LCD) or an organic EL display. The display device 192 displays various screens under the control of the control unit 10. This screen may include various screens, such as a display screen related to reagent amount, which will be described later, an operation screen of the automatic analysis device 1, a screen showing measurement results, and a screen showing analysis results. The touch panel 194 is provided above the display device 192. The touch panel 194 acquires input from a user and transmits obtained input information to the control unit 10.

The control unit 10 may be connected to other devices such as a printer, a handy code reader, and a host computer via the communication I/F 20.

The measurement unit 30 includes a control circuit 42, a data processing circuit 44, a constant-temperature bath 52, the reaction vessel 54, a light source 62, a scattered light detector 64, a transmitted light detector 66, a sample vessel 72, the reagent vessel 74, a sample probe 76, and a reagent probe 78. In this embodiment, as an example, the reaction vessel 54, the scattered light detector 64, and the transmitted light detector 66 are provided in the constant-temperature bath 52. However, the invention is not limited to such an arrangement form.

The control circuit 42 controls operations of respective parts of the measurement unit 30 based on commands from the control unit 10. Although not illustrated, the control circuit 42 is connected to the data processing circuit 44, the constant-temperature bath 52, the light source 62, the scattered light detector 64, the transmitted light detector 66, the sample probe 76, the reagent probe 78, etc., and controls operations of respective parts.

The data processing circuit 44 is connected to the scattered light detector 64 and the transmitted light detector 66, and acquires detection results from the scattered light detector 64 and the transmitted light detector 66. The data processing circuit 44 performs various processing on the acquired detection results and outputs processing results. The processing performed by the data processing circuit 44 may include, for example, A/D conversion processing for changing a format of data output from the scattered light detector 64 and the transmitted light detector 66 into a format that can be processed by the control unit 10.

The control circuit 42 and the data processing circuit 44 may include, for example, a CPU, an application specific integrated circuit (ASIC), a field programmable gate array (FPGA), etc. The control circuit 42 and the data processing circuit 44 may each be configured by one integrated circuit, etc., or may be configured by combining a plurality of integrated circuits, etc. Furthermore, the control circuit 42 and the data processing circuit 44 may be configured by one integrated circuit, etc. For example, operations of the control circuit 42 and the data processing circuit 44 may be performed according to a program recorded in a storage device or a recording area in the circuit.

For example, a sample obtained from blood collected from a patient is accommodated in the sample vessel 72. Various reagents used for measurement are accommodated in the reagent vessel 74. Any number of sample vessels 72 and reagent vessels 74 may be provided. Since there is usually a plurality of types of reagents used for analysis, there is generally a plurality of reagent vessels 74. The sample probe 76 dispenses the sample accommodated in the sample vessel 72 into the reaction vessel 54 under the control of the control circuit 42. The reagent probe 78 dispenses the reagent accommodated in the reagent vessel 74 into the reaction vessel 54 under the control of the control circuit 42. The number of sample probes 76 and reagent probes 78 may be any number.

The constant-temperature bath 52 maintains a temperature of the reaction vessel 54 at a predetermined temperature under the control of the control circuit 42. In the reaction vessel 54, a mixed liquid obtained by mixing the sample dispensed by the sample probe 76 and the reagent dispensed by the reagent probe 78 reacts. Note that there may be any number of reaction vessels 54.

The light source 62 radiates light of a predetermined wavelength under the control of the control circuit 42. The light source 62 may be configured to radiate light having different wavelengths depending on the measurement condition. Therefore, the light source 62 may include a plurality of light source elements. Light radiated from the light source 62 is guided by, for example, an optical fiber, and is radiated onto the reaction vessel 54. A part of light radiated onto the reaction vessel 54 is scattered and another part thereof penetrates depending on the reaction process state of the mixed liquid in the reaction vessel 54. The scattered light detector 64 detects light scattered by the reaction vessel 54.

The transmitted light detector 66 detects light transmitted through the reaction vessel 54. The data processing circuit 44 processes information on the amount of scattered light detected by the scattered light detector 64 and information on the amount of transmitted light detected by the transmitted light detector 66. Either the scattered light detector 64 or the transmitted light detector 66 may operate depending on the measurement condition. Therefore, the data processing circuit 44 may process either the information on the amount of scattered light detected by the scattered light detector 64 or the information on the amount of transmitted light detected by the transmitted light detector 66, depending on the measurement condition. The data processing circuit 44 transmits processed data to the control unit 10. Note that, even though the measurement unit 30 illustrated in Fig. 2 includes two detectors of the scattered light detector 64 and the transmitted light detector 66, the measurement unit 30 may include only one of the detectors.

The control unit 10 performs various calculations based on data acquired from the measurement unit 30. The calculations include calculation of the reaction amount of the mixed liquid, quantitative calculation of the substance amount and an activity value of a substance to be measured in a test object based on the reaction amount, etc. The data processing circuit 44 may perform some or all of these calculations.

Note that, here, a case in which a PC that controls an operation of the measurement unit 30 and a PC that performs data calculation and quantitative calculation are the same control unit 10 is illustrated. However, these PCs may be separate units. In other words, the PC that performs data calculation and quantitative calculation may exist as each.

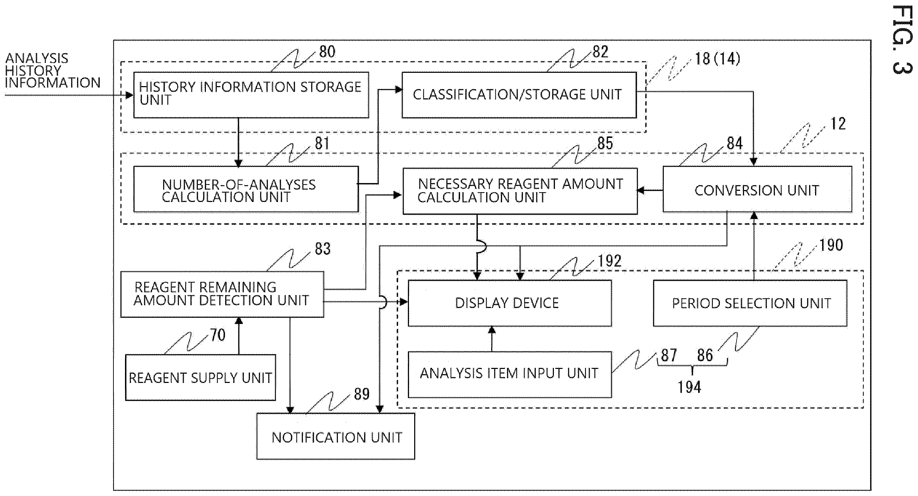

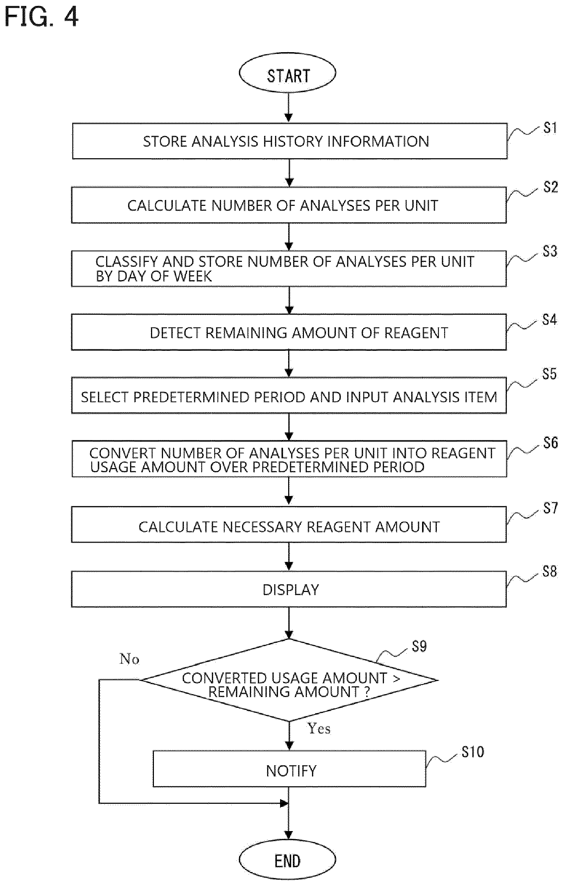

Next, an example of each of a configuration for realizing a reagent amount display method and a reagent amount display method procedure according to an embodiment of the invention will be described with reference to Figs. 3 and 4.

As illustrated in Fig. 3, the automatic analysis device 1 of this embodiment includes a history information storage unit 80 that stores history information related to a previously conducted analysis and is transmitted from the measurement unit 30 via the communication I/F 20, a number-of-analyses calculation unit 81 that calculates the number of analyses per unit, which is the number of analyses per day on each analysis day, for each analysis item from the history information stored in the history information storage unit 80, a classification/storage unit 82 that classifies the number of analyses per unit calculated by the number-of-analyses calculation unit 81 by day of the week and stores the number of analyses per unit, a reagent remaining amount detection unit 83 that detects the current remaining amount of each reagent in the above-mentioned reagent supply unit 70, a conversion unit 84 that reads each number of analyses per unit over a predetermined period corresponding to a day of the week of an analysis implementation date from the classification/storage unit 82 and converts the read each number of analyses per unit into the reagent usage amount with regard to each reagent used for the analysis for each analysis item, and a necessary reagent amount calculation unit 85 that calculates a necessary reagent amount with which the reagent supply unit 70 is replenished based on the remaining amount of the reagent detected by the reagent remaining amount detection unit 83 and the reagent usage amount converted by the conversion unit 84.

Among these components, at least the history information storage unit 80, the number-of-analyses calculation unit 81, the classification/storage unit 82, the conversion unit 84, and the necessary reagent amount calculation unit 85 are included in the control unit 10. In particular, in this embodiment, the history information storage unit 80 and the classification/storage unit 82 may be configured by the RAM 14 and/or the storage 18, and the number-of-analyses calculation unit 81, the conversion unit 84, and the necessary reagent amount calculation unit 85 may be configured by the CPU 12.

In addition, data related to the reagent remaining amount detected by the reagent remaining amount detection unit 83, data related to the reagent usage amount converted by the conversion unit 84, and data related to the necessary reagent amount calculated by the necessary reagent amount calculation unit 85 are transmitted to the display device 192 of the touchscreen 190 without change or by being converted into related data (this conversion may previously be performed by the conversion unit 84, or the number of analyses per unit may be directly converted into data related to the reagent usage amount without the conversion unit 84 performing conversion), and the display device 192 arranges and displays information associated with the reagent usage amount, the reagent remaining amount, and the necessary reagent amount for each reagent (analysis item) as described later.

Further, the data related to the reagent remaining amount detected by the reagent remaining amount detection unit 83 and the data related to the reagent usage amount converted by the conversion unit 84 are further transmitted to a notification unit 89, and when the remaining amount of each reagent detected by the reagent remaining amount detection unit 83 is smaller than each reagent usage amount converted by the conversion unit 84, the notification unit 89 notifies this information.

In addition, the automatic analysis device 1 of this embodiment includes a period selection unit 86 for selecting the predetermined period (predetermined period corresponding to the day of the week of the analysis implementation date) of the number of analyses per unit to be read by the conversion unit 84 from the classification/storage unit 82, and an analysis item input unit 87 for inputting an analysis item to be executed by the measurement unit 30. In this case, the period selection unit 86 and the analysis item input unit 87 are configured by, for example, the touch panel 194 of the touchscreen 190 in this embodiment.

In addition, when information related to the reagent amount is displayed based on such a configuration, first, the premise is that history information related to a previously conducted analysis is stored in the history information storage unit 80 (history information storage step S1 of Fig. 4). Then, automatically when the power of the automatic analysis device 1 is turned on, or based on predetermined input through the touch panel 194, the number-of-analyses calculation unit 81 of the CPU 12 calculates the number of analyses per unit, which is the number of analyses per day on each analysis day, for each analysis item from the history information stored in the history information storage unit 80 (in history information storage step S 1) (number-of-analyses calculation step S2 of Fig. 4).

Thereafter, the classification/storage unit 82 of the CPU 12 classifies and stores the number of analyses per unit calculated in the number-of-analyses calculation unit 81 (number-of-analyses calculation step S2) by day of the week (classification storage step S3 of Fig. 4). During this time or subsequently, the reagent remaining amount detection unit 83 detects the current remaining amount of each reagent in the reagent supply unit 70 (reagent remaining amount detection step S4 of Fig. 4), and an operator selects the predetermined period of the number of analyses per unit to be read by the conversion unit 84 from the classification/storage unit 82 through the period selection unit 86 of the touch panel 194 (the predetermined period can be set arbitrarily or from selectable units such as 1 week, 1 month, and 3 months), and inputs an analysis item to be executed by the measurement unit 30 through the analysis item input unit 87 of the touch panel 194 (period selection/analysis item input step S5 of Fig. 4).

Then, the conversion unit 84 of the CPU 12 reads each number of analyses per unit over the predetermined period corresponding to the day of the week of the analysis implementation date from the classification/storage unit 82, and converts the read each number of analyses per unit into the reagent usage amount with regard to each reagent used for the analysis for each analysis item (conversion step S6 of Fig. 4). Thereafter, the necessary reagent amount calculation unit 85 calculates the necessary reagent amount with which the reagent supply unit 70 is replenished based on the remaining amount of the reagent detected by the reagent remaining amount detection unit 83 and the reagent usage amount converted by the conversion unit 84 (necessary reagent amount calculation step S7 of Fig. 4).

In this way, the data related to the reagent remaining amount detected by the reagent remaining amount detection unit 83, the data related to the reagent usage amount converted by the conversion unit 84, and the data related to the necessary reagent amount calculated by the necessary reagent amount calculation unit 85 are transmitted to the display device 192 of the touchscreen 190, and the display device 192 arranges and displays reagent amount-associated information including data associated with the reagent usage amount, the reagent remaining amount, and the necessary reagent amount for each reagent (analysis item) (display step S8 of Fig. 4).

In addition, especially in this embodiment, along with this display or independently of the display, the notification unit 89 or the CPU 12 that controls the notification unit 89 determines whether or not the remaining amount of each reagent detected by the reagent remaining amount detection unit 83 (reagent remaining amount detection step S4) is smaller than each reagent usage amount converted by the conversion unit (conversion step) (step S9 of Fig. 4), and when the remaining amount of the reagent is smaller than the converted reagent usage amount, the notification unit 89 notifies this information (notification step S 10 of Fig. 4).

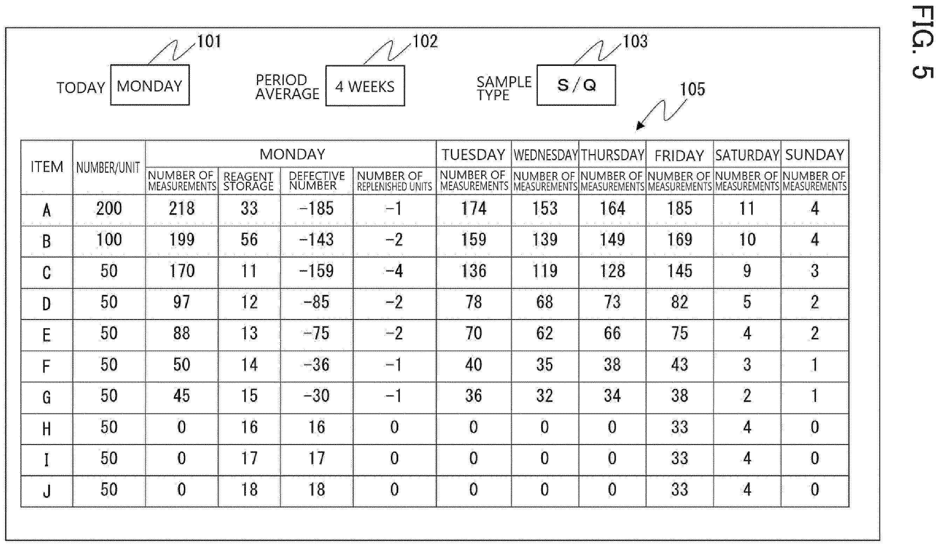

Fig. 5 illustrates an example of a display mode of the reagent amount-associated information by the display device 192. As illustrated in the figure, in this display, the day of the week "today" 101 (analysis implementation date; Monday on this screen) selected through the touch panel 194 including the period selection unit 86, a period average 102 (predetermined period (predetermined period corresponding to the day of the week of the analysis implementation date) of the number of analyses per unit to be read by the conversion unit 84 from the classification/storage unit 82; in this screen, 4 weeks), and a sample type 103 (in this screen, patient sample (S) and QC (quality control) sample (Q)) are displayed at the top of the screen.

In addition, information associated with the reagent usage amount converted by the conversion unit 84, here, the "number of measurements" for each day of the week (in this example where both the patient sample and the QC sample are selected, the average number of measurements obtained by summing the patient sample and the QC sample), and the number of measurements per reagent vessel "number/unit" is arranged and displayed in a main area 105 of a display screen for each analysis item (or reagent; in this screen, analysis items A to J). In this display example, the analysis items A to J input through the analysis item input unit 87 are displayed in a vertical column on the display screen, and the "number of measurements", etc. for each day of the week are displayed in a horizontal column. It is obvious that the measurement amount (mL) may be displayed instead of the number of measurements.

As described above, the display device 192 arranges and displays information associated with each reagent usage amount converted by the conversion unit 84 and the remaining amount of each reagent detected by the reagent remaining amount detection unit 83 for each reagent (analysis item) with regard only to an analysis item input by the analysis item input unit 87. However, display may be performed with regard to an analysis item other than the analysis item input by the analysis item input unit 87.

Further, in this display example, with regard to the selected day of the week "Today" (Monday on this screen), not only the "number of measurements" but also information associated with the current remaining amount of each reagent in the reagent supply unit (reagent storage) 70, here, the number of remaining measurements of the reagent installed in the reagent storage (when the number of remaining measurements is different in a 2-reagent system that uses two reagents for one analysis item, the smaller number of remaining measurements) "reagent storage" and the "defective number" obtained by subtracting the number of remaining measurements from the number of measurements ("number of measurements" - "reagent storage"), and information associated with the necessary reagent amount with which the reagent supply unit (reagent storage) 70 is replenished, here, the number of the reagent vessels 74 to be replenished "the number of replenished units", that is, the number obtained by multiplying the "defective number" by "number/unit" (the quotient is rounded up) are further displayed. When replenished with the reagent, the latest reagent amount state after replenishment may be automatically updated and displayed.

Further, in this display example, an information item associated with the selected day of the week (Monday in this screen) is shaded or colored so as to be visually distinguished from other days of the week. Furthermore, when there is a shortage of reagent, that is, when a numerical value on the display screen is negative (-), the numerical value is visually distinguished (for example, colored in red, etc.) and displayed.

Note that, in this embodiment, the number-of-analyses calculation unit 81 calculates a reanalysis frequency with regard to each reagent used for the analysis for each analysis item, and the display device 192 may display information on the reanalysis frequency. In addition, the necessary reagent amount calculation unit 85 preferably calculates the necessary reagent amount only for the analysis item input by the analysis item input unit 87 as an analysis item necessary for an analysis of the day at a calculation time point, so that calculation processing can be reduced.

Further, the classification/storage unit 82 may classify the number of analyses per unit as the number of calibrator measurements, the number of accuracy management sample measurements, the number of initial tests, the number of retests, and the total number thereof and store the number of analyses per unit. In this case, the conversion unit 84 converts each of the number of calibrator measurements, the number of accuracy management sample measurements, the number of initial tests, the number of retests, and the total number thereof read from the classification/storage unit 82 into the reagent usage amount with regard to each reagent used for the analysis for each analysis item. Further, with regard thereto, the number-of-analyses calculation unit 81 may calculate the number of analyses per unit by subtracting the number of calibrator measurements and/or the number of accuracy management sample measurements from the analysis item.

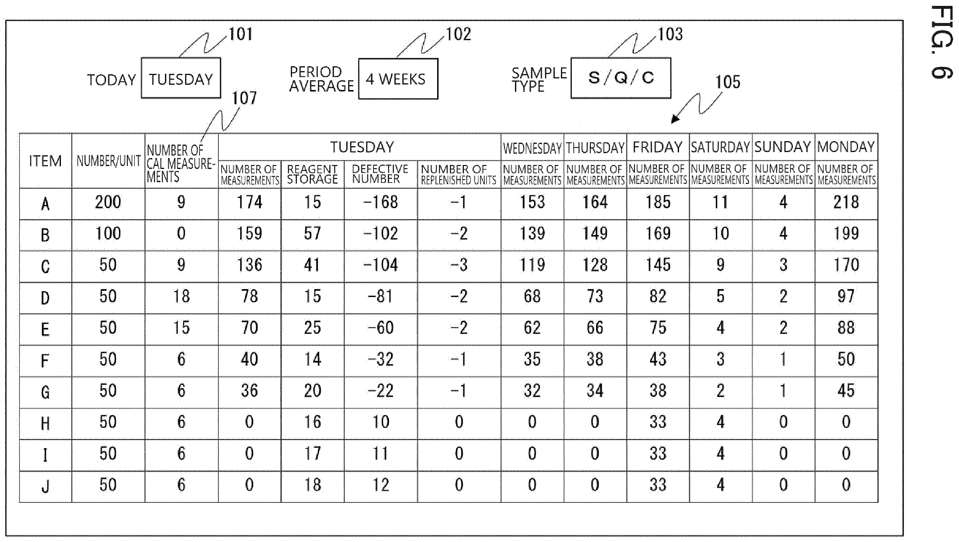

An example of a display screen including the number of calibrator measurements is illustrated in Fig. 6. As illustrated in the figure, in this display example, Tuesday is selected as the day of the week "today" 101 indicating the analysis implementation date. Therefore, as not only "the number of measurements" but also the number of remaining measurements of the reagent "reagent storage", the "defective number", and the "number of replenished units" are further displayed with regard only to Tuesday, and patient sample (S), QC sample (Q), and calibrator (C) are selected as the sample type 103, the number of calibrator measurements "number of CAL measurements" 107, that is, the number of measurements necessary for calibration is newly displayed in the main area 105 of the display screen in addition to the information item described with regard to Fig. 5.

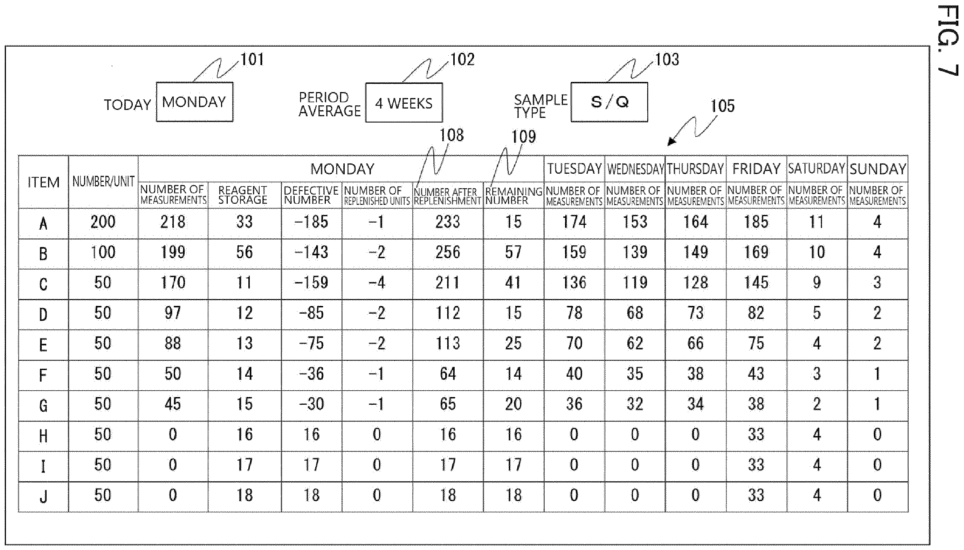

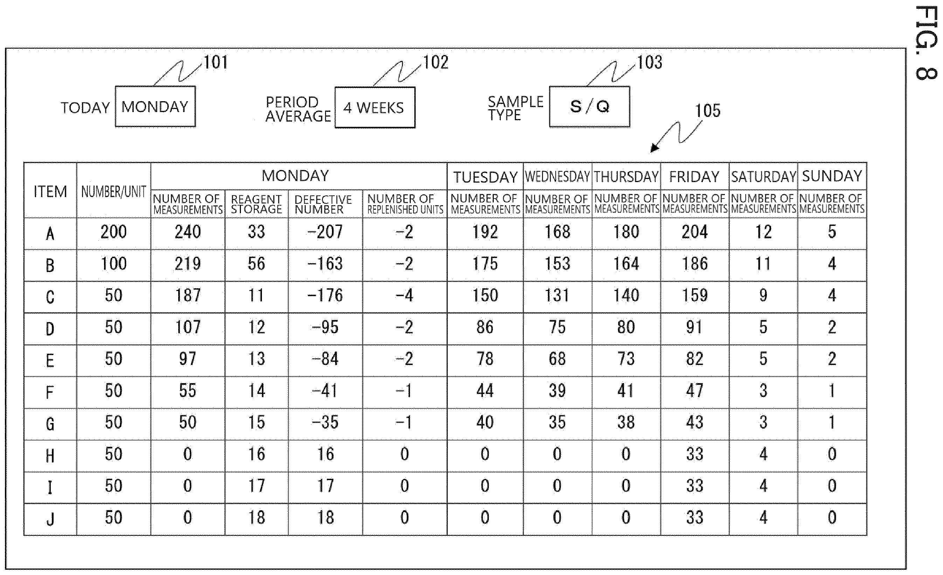

Fig. 7 illustrates a first modification of the display mode of Fig. 5. As illustrated in the figure, in this display example, the "number after replenishment" 108 which is the number of measurements when replenished with the reagent according to the "number of replenished units" and the "remaining number" 109 which is the number of measurements remaining when replenished with the reagent in this way are newly displayed in addition to the information item described with regard to Fig. 5. In addition, Fig. 8 illustrates a second modification of the display mode of Fig. 5. As illustrated in the figure, in this display example, a maximum value on the same day of the week (here, Monday as in Fig. 5) within the selected predetermined period is displayed as the "number of measurements." Therefore, numerical values of the "defective number" and the "number of replenished units" are increased when compared to Fig. 5.

As described above, according to this embodiment, the number of analyses per unit, which is the number of analyses per day on each analysis day, is calculated for each analysis item from the history information related to the previously conducted analysis, the calculated number of analyses per unit is classified by day of the week, each number of analyses per unit over the predetermined period corresponding to the day of the week of the implementation analysis date is converted into the reagent usage amount with regard to each reagent used for the analysis for each analysis item, and information related to the converted each reagent usage amount and the current remaining amount of each reagent in the reagent supply unit 70 is arranged and displayed for each reagent. Therefore, according to this display, even an inexperienced operator easily understands a difference between the estimated (or required) reagent amount necessary on the day (day of the week) and the reagent amount previously provided in the automatic analysis device 1. Therefore, an appropriate amount of reagent can be maintained at all times by replenishment with at least the reagent of the amount corresponding to the difference before starting to analyze the analysis implementation date. For this reason, it is possible to avoid inconvenience of running out of reagent during analysis to force the analysis to be interrupted, thereby causing such as wasting the sample being analyzed.

### EXPLANATIONS OF LETTERS OR NUMERALS

- 1: AUTOMATIC ANALYSIS DEVICE
- 40: REACTION UNIT
- 54: REACTION VESSEL
- 70: REAGENT SUPPLY UNIT
- 80: HISTORY INFORMATION STORAGE UNIT
- 81: NUMBER-OF-ANALYSES CALCULATION UNIT
- 82: CLASSIFICATION/STORAGE UNIT
- 83: REAGENT REMAINING AMOUNT DETECTION UNIT
- 84: CONVERSION UNIT
- 85: NECESSARY REAGENT AMOUNT CALCULATION UNIT
- 86: PERIOD SELECTION UNIT
- 87: ANALYSIS ITEM INPUT UNIT
- 89: NOTIFICATION UNIT
- 192: DISPLAY DEVICE (DISPLAY UNIT)

## Claims

1. An automatic analysis device including a reaction unit configured to hold a reaction vessel into which a sample collected from a person such as blood or urine is dispensed, and a reagent supply unit configured to supply a reagent, and obtaining measurement information with regard to a predetermined analysis item by measuring a test liquid obtained by mixing and reacting the reagent supplied from the reagent supply unit to the reaction vessel with the sample, the automatic analysis device comprising:
a history information storage unit configured to store history information related to a previously conducted analysis;
a number-of-analyses calculation unit configured to calculate a number of analyses per unit, the number of analyses per unit being a number of analyses per day on each analysis day, for each analysis item from the history information stored in the history information storage unit;
a classification/storage unit configured to classify the number of analyses per unit calculated by the number-of-analyses calculation unit for each day of a week and store the number of analyses per unit;
a reagent remaining amount detection unit configured to detect a current remaining amount of each reagent in the reagent supply unit;
a conversion unit configured to read each number of analyses per unit over a predetermined period corresponding to a day of a week of an analysis implementation date from the classification/storage unit and convert the read each number of analyses per unit into a reagent usage amount with regard to each reagent used for the analysis for each analysis item; and
a display unit configured to arrange and display information associated with each reagent usage amount converted by the conversion unit and a remaining amount of each reagent detected by the reagent remaining amount detection unit for each reagent.

2. The automatic analysis device according to claim 1, further comprising a period selection unit for selection of the predetermined period.

3. The automatic analysis device according to claim 1 or 2, wherein:
the classification/storage unit classifies the number of analyses per unit as a number of calibrator measurements, a number of accuracy management sample measurements, a number of initial tests, a number of retests, and a total number thereof and stores the number of analyses per unit, and
the conversion unit converts each of the number of calibrator measurements, the number of accuracy management sample measurements, the number of initial tests, the number of retests, and the total number thereof read from the classification/storage unit into a reagent usage amount with regard to each reagent used for the analysis for each analysis item.

4. The automatic analysis device according to any one of claims 1 to 3, wherein the number-of-analyses calculation unit calculates the number of analyses per unit by subtracting the number of calibrator measurements and/or the number of accuracy management sample measurements from the analysis item.

5. The automatic analysis device according to any one of claims 1 to 4, wherein the number-of-analyses calculation unit calculates a reanalysis frequency with regard to each reagent used for the analysis for each analysis item, and the display unit displays information on the reanalysis frequency.

6. The automatic analysis device according to any one of claims 1 to 5, further comprising a notification unit configured to notify information indicating that the remaining amount of each reagent detected by the reagent remaining amount detection unit is smaller than each reagent usage amount converted by the conversion unit when the remaining amount of each reagent detected by the reagent remaining amount detection unit is smaller than each reagent usage amount converted by the conversion unit.

7. The automatic analysis device according to any one of claims 1 to 6, further comprising an analysis item input unit for input of an analysis item to be executed,
wherein the display unit arranges and displays the information associated with each reagent usage amount converted by the conversion unit and the remaining amount of each reagent detected by the reagent remaining amount detection unit for each reagent exclusively with regard to the analysis item input by the analysis item input unit.

8. A reagent amount display method in an automatic analysis device including a reaction unit configured to hold a reaction vessel into which a sample collected from a person such as blood or urine is dispensed, and a reagent supply unit configured to supply a reagent, and obtaining measurement information with regard to a predetermined analysis item by measuring a test liquid obtained by mixing and reacting the reagent supplied from the reagent supply unit to the reaction vessel with the sample, the method comprising:
a history information storage step of storing history information related to a previously conducted analysis;
a number-of-analyses calculation step of calculating a number of analyses per unit, the number of analyses per unit being a number of analyses per day on each analysis day, for each analysis item from the history information stored in the history information storage step;
a classification/storage step of classifying the number of analyses per unit calculated in the number-of-analyses calculation step for each day of a week and storing the number of analyses per unit in a storage unit;
a reagent remaining amount detection step of detecting a current remaining amount of each reagent in the reagent supply unit;
a conversion step of reading each number of analyses per unit over a predetermined period corresponding to a day of a week of an analysis implementation date from the storage unit and converting the read each number of analyses per unit into a reagent usage amount with regard to each reagent used for the analysis for each analysis item; and
a display step of arranging and displaying information associated with each reagent usage amount converted by the conversion step and a remaining amount of each reagent detected by the reagent remaining amount detection step for each reagent.

9. The reagent amount display method according to claim 8, further comprising a period selection step of selecting the predetermined period.

10. The reagent amount display method according to claim 8 or 9, wherein:
the classification/storage step classifies the number of analyses per unit as a number of calibrator measurements, a number of accuracy management sample measurements, a number of initial tests, a number of retests, and a total number thereof and stores the number of analyses per unit, and
the conversion step converts each of the number of calibrator measurements, the number of accuracy management sample measurements, the number of initial tests, the number of retests, and the total number thereof read from the storage unit into a reagent usage amount with regard to each reagent used for the analysis for each analysis item.

11. The reagent amount display method according to any one of claims 8 to 10, wherein the number-of-analyses calculation step calculates the number of analyses per unit by subtracting the number of calibrator measurements and/or the number of accuracy management sample measurements from the analysis item.

12. The reagent amount display method according to any one of claims 8 to 11, wherein the number-of-analyses calculation step calculates a reanalysis frequency with regard to each reagent used for the analysis for each analysis item, and the display step displays information on the reanalysis frequency.

13. The reagent amount display method according to any one of claims 8 to 12, further comprising a notification step of notifying information indicating that the remaining amount of each reagent detected by the reagent remaining amount detection step is smaller than each reagent usage amount converted by the conversion step when the remaining amount of each reagent detected by the reagent remaining amount detection step is smaller than each reagent usage amount converted by the conversion step.

14. The reagent amount display method according to any one of claims 8 to 13, further comprising an analysis item input step of inputting of an analysis item to be executed,
wherein the display step arranges and displays the information associated with each reagent usage amount converted by the conversion step and the remaining amount of each reagent detected by the reagent remaining amount detection step for each reagent exclusively with regard to the analysis item input in the analysis item input step.

15. The reagent amount display method according to claim 14, further comprising a necessary reagent amount calculation step of calculating a necessary reagent amount with which the reagent supply unit is replenished based on the remaining amount of the reagent detected by the reagent remaining amount detection step and the reagent usage amount converted by the conversion step,
wherein the necessary reagent amount calculation step calculates the necessary reagent amount exclusively for the analysis item input in the analysis item input step as an analysis item necessary for an analysis of a day at a calculation time point.
